# EUROPEAN PATENT SPECIFICATION

(11) **EP 4 249 887 B1**
(45) Date of publication and mention of the grant of the patent: **09.12.2026**
(21) Application number: 23160436.4
(22) Date of filing: 07.03.2023
(51) Int. Cl.: G01N 1/22, G01N 33/00, G01N 1/24, G01N 15/06

(54) **DEVICE FOR DETECTING GAS AND CONTROLLING VOLUME OF CLEAN GAS**
VORRICHTUNG ZUR DETEKTION VON GAS UND ZUR STEUERUNG DES VOLUMENS VON REINGAS
DISPOSITIF DE DÉTECTION DE GAZ ET DE RÉGULATION DU VOLUME DE GAZ PROPRE

(30) Priority: 25.03.2022 TW 111111370
(43) Date of publication of application: 27.09.2023
(73) Proprietor: Microjet Technology Co., Ltd., Hsinchu (TW)
(72) Inventor: Mou, Hao-Jan, Hsinchu (TW); Wu, Chin-Chuan, Hsinchu (TW); Han, Yung-Lung, Hsinchu (TW); Huang, Chi-Feng, Hsinchu (TW)
(74) Representative: Uexküll & Stolberg

(56) References cited:
- CN-A- 109 751 735
- CN-A- 113 075 096
- CN-U- 204 593 760
- US-A1- 2022 057 091

## Description

### FIELD OF THE INVENTION

The present disclosure relates to a device for controlling a volume of a clean gas, and more particularly to a device for controlling the volume of the clean gas according to a gas detection.

### BACKGROUND OF THE INVENTION

In recent years, people pay more and more attention to the air quality around their living environment. Gases, such as volatile organic compounds (VOC) and particulate matter 2.5 (PM2.5), and even suspended particles contained in the air which are exposed in the environment might affect human health, and even endanger people's life in severe condition. Therefore, the quality of the ambient air has attracted more attentions in various countries, and how to monitor and avoid the harm is an urgent topic.

It is known to a gas detector can be utilized to monitor the ambient air for determining the air quality. Accordingly, if the monitoring information can be provided in real time to warn people in the harmful environment, people can take a precaution or get away immediately, so as to avoid from the health impact and injury caused by exposing to harmful gases in the environment. Thus, utilizing the gas detector to monitor the ambient air is an appropriate and well application. Further, it is also known that the air purification device is an air pollution solution for modern people to prevent from inhaling harmful gases. Therefore, the main subject of the present disclosure is to combine the air purification device and the gas detector, so that the air quality can be monitored in real time, and the volume of clean gas from the air purification device can be controlled according to the monitored air quality, thereby achieving a best benefit of improving and purifying the air quality near a user.

US 2022/057091 A1 relates to a purification device for exercise environment which includes a main body, a purification unit, a gas guider and a gas detection module. The purification unit, the gas guider and the gas detection module are disposed in the main body to guide the gas outside the main body through the purification unit for filtering and purifying the gas, and discharge a purified gas. The gas detection module detects particle concentration of suspended particles contained in the purified gas. The gas guider is controlled to operate and export the gas at an airflow rate within 3 minutes. The particle concentration of the suspended particles contained in the purified gas, which is filtered by the purification unit, is reduced to and less than 0.75 µg/m³. Consequently, the purified gas is filtered, and an exerciser in an exercise environment can breathe with safety.

CN 204 593 760 U relates to an intelligence air purifier, wherein the clarifier includes filtration system, air exchange system, clean system, intelligent air detecting system, communication module, control module and orientation module, including detection module among the intelligent air detecting system, wherein, detection module detects the air quality to send the information that detects for the customer end with the help of wireless communication network and server through communication module, carry out real time monitoring and look over by this customer end, this customer end is sent control information for the remote control of control module realization to air purifier with the help of wireless communication network and server through communication module. Through the utility model discloses, realize air purifier's remote control, improve user experience.

CN 109 751 735 A provides a fresh air control system which includes a detector and a fan intercommunicating with each other. The fan includes a main control module, a temperature sensing assembly, an air inlet valve, an air regulator, a heater and a communication module, wherein the temperature sensing assembly communicates with the main control module, the air inlet valve communicates with the main control module, the air regulator communicates with the main control module, the heater communicates with the main control module, the communication module communicates with the main control module, and the fan intercommunicates with the detector through the communication module.

Patent application CN113075096 discloses a gas detection and purification remote control system, comprising a first gas detection module and a second gas detection module, the second gas detection module detects the gas at the location where the gas purification devices are located, and provides and outputs a second gas detection data. The second gas detection data detected and output by the second gas detection module of the gas purification device is wirelessly transmitted to an external connection device.

Compared with the prior art, the fresh air control system has the following advantages that 1, a fresh air ventilator system is separated from an air detection device, air quality monitoring can be conducted aiming at targeted areas, rather than just by the side of fresh air ventilators, and the great use value is achieved for the fresh air ventilator system of the whole house site; and 2, operation of the fresh air ventilator system can be controlled according to the ambient air quality as input, and the purpose of intelligent control is realized through a configurable threshold and policy setting.

### SUMMARY OF THE INVENTION

The object of the present disclosure is to provide a device for detecting gas and controlling volume of clean gas, in which a gas detector is combined with an air purification device, so that the air quality can be monitored in real time, and the volume of clean gas from the air purification device can be controlled according to the monitored air quality, thereby achieving a best benefit of improving and purifying the air quality near a user.

The invention is set out in the appended set of claims.

### BRIEF DESCRIPTION OF THE DRAWINGS

The above contents of the present disclosure will become more readily apparent to those ordinarily skilled in the art after reviewing the following detailed description and accompanying drawings, in which:
FIG. 1 is a circuit block illustrating the driving and controlling circuitry for controlling a volume of clean gas according to an embodiment of the present disclosure;
FIG. 2 is a schematic view illustrating a device for detecting gas and controlling volume of clean gas according to an example useful to understand the invention;
FIG. 3 is a schematic view illustrating the device for detecting gas and controlling volume of clean gas according to the invention of the present disclosure;
FIG. 4 is a schematic view illustrating the structure of an air purification device according to an embodiment of the present disclosure;
FIG. 5 is a schematic view illustrating the appearance of a gas detection module according to an embodiment of the present disclosure;
FIG. 6A is a schematic exploded view illustrating a gas detection main part according to an embodiment of the present disclosure;
FIG. 6B is a schematic view illustrating a base of the gas detection main part according to the embodiment of the present disclosure;
FIG. 6C is a schematic view illustrating the base of the gas detection main part according to the embodiment of the present disclosure from another view angle;
FIG. 7 is a schematic view illustrating the base of the gas detection main part with a laser component and a particulate sensor accommodated therein according to an embodiment of the present disclosure;
FIG. 8A is a schematic exploded view illustrating a piezoelectric actuator of the gas detection main part combining with the base according to an embodiment of the present disclosure;
FIG. 8B is a schematic view illustrating the piezoelectric actuator of the gas detection main part combined with the base according to the embodiment of the present disclosure;
FIG. 9 is a schematic exploded view illustrating the piezoelectric actuator of the gas detection main part according to the embodiment of the present disclosure;
FIG. 10A is a schematic view illustrating the piezoelectric actuator of the gas detection main part combined in a gas-guiding-component loading region according to an embodiment of the present disclosure;
FIG. 10B and FIG. 10C are schematic views illustrating operation steps of the piezoelectric actuator in FIG. 10A;
FIG. 11A to FIG. 11C are schematic views illustrating a gas flowing path of the gas detection main part according to an embodiment of the present disclosure; and
FIG. 12 is a schematic view illustrating a position of a gas sensor in the gas detection main part and a light beam path emitted from the laser component according to an embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this disclosure are presented herein for purpose of illustration and description only. It is not intended to be exhaustive or to be limited to the precise form disclosed.

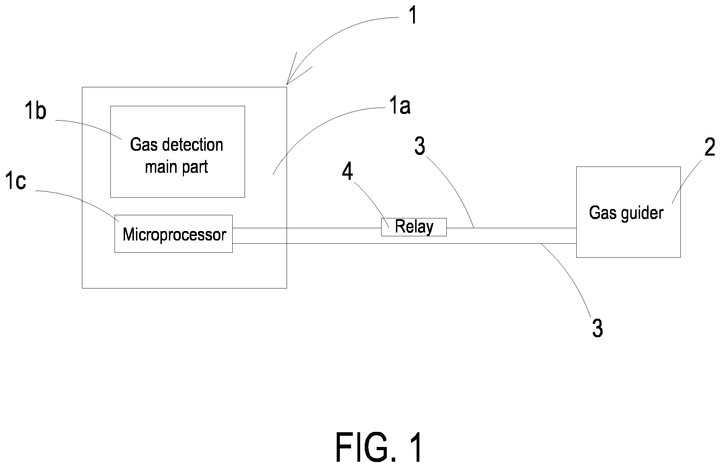

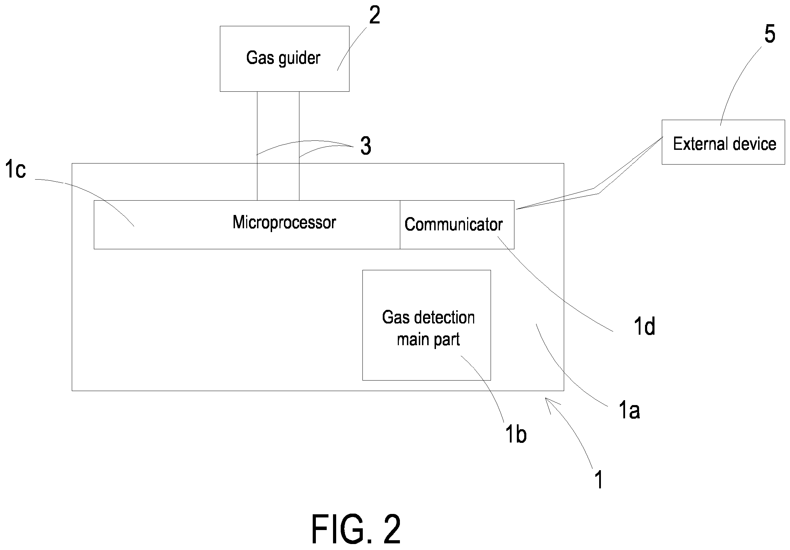

Please refer to FIG. 1 and FIG. 2. The present disclosure provides a device for detecting gas and controlling volume of clean gas including a gas detection module 1 and at least one gas guider 2. The gas detection module 1 detects a gas to generate a gas detection data and converts the gas detection data into at least one power signal and a PWM (Pulse-Width Modulation) signal for outputting to the gas guider 2. Accordingly, based on the gas detection data, the gas guider 2 is enabled or disabled, and a rotation speed of the gas guider 2 is adjusted for changing a volume of clean gas therefrom.

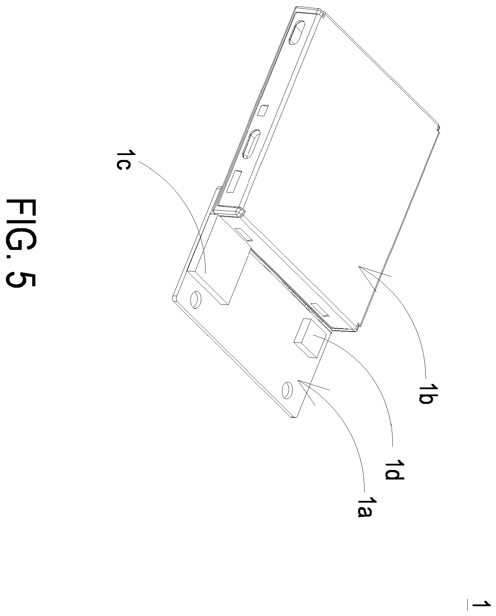

The gas detection module 1 mentioned above includes a control circuit board 1a, a gas detection main part 1b and a microprocessor 1c. The gas detection main part 1b and the microprocessor 1c are integrally packaged on and electrically connected with the control circuit board 1a (as shown in FIG. 5). The gas detection main part 1b detects a gas to generate a signal and transmits the signal to the microprocessor 1c for calculating and processing so as to generate a gas detection data. Base on the gas detection data, the microprocessor 1c further outputs at least one power signal and a PWM signal. The gas guider 2 receives the least one power signal and the PWM signal outputted from the microprocessor 1c, and based thereon, the gas guider 2 is enabled or disabled and a rotation speed of the gas guider 2 and also the output volume of clean gas therefrom are adjusted. The microprocessor 1c is electrically connected to the gas guider 2 through at least one wire 3 and also transmits the power signal and the PWM signal to the gas guider 2 through the wire 3. A relay 4 is further disposed on the above-mentioned wire 3 to form a buffer switch so as to achieve a voltage protection for the microprocessor 1c. In one embodiment, the gas guider 2 is a direct current (DC) fan.

As shown in FIG. 2 (an example useful to understand the invention), the gas detection module 1 further includes a communicator 1d which is integrally packaged on and electrically connected with the control circuit board 1a (as shown in FIG. 5). The microprocessor 1c receives, calculates and processes the signal generated after detecting the gas so as to generate a gas detection data for providing to the communicator 11d to perform an external transmission. The communicator 11d transmits the gas detection data externally to an external device 5, and the external device 5 receives and displays the gas detection data, and accordingly, generates a notification alert. The external device 5 is a mobile device.

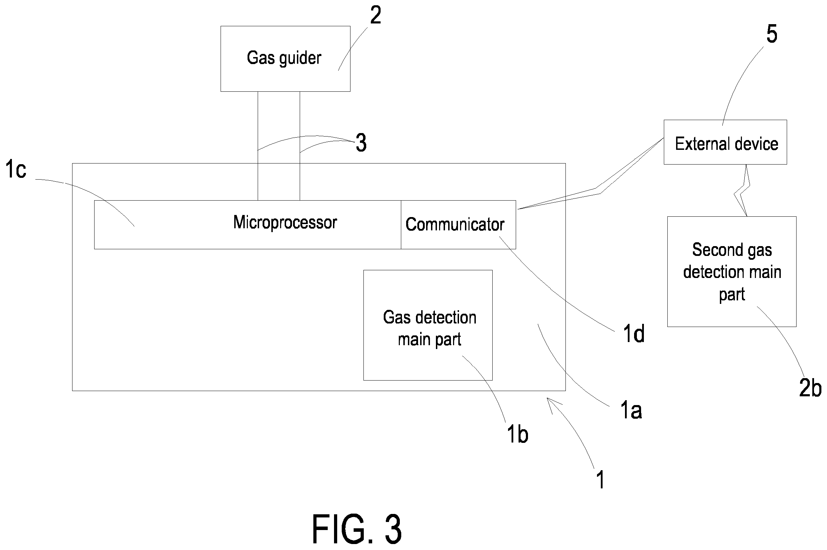

Please refer to FIG. 3. In the present invention, the device for detecting gas and controlling volume of clean gas of the present disclosure further includes at least one second gas detection main part 2b. The second gas detection main part 2b is provided for generating and outputting another gas detection data to the external device 5. Then, the external device 5 receives the another gas detection data and transmits thereof to the microprocessor 1c via the communicator 11d. The microprocessor 11c receives, calculates and processes the another gas detection data for outputting the power signal and the PWM signal to the gas guider 2, so as to enable or disable the gas guider 2 and adjust the rotation speed thereof and the output volume of clean gas therefrom.

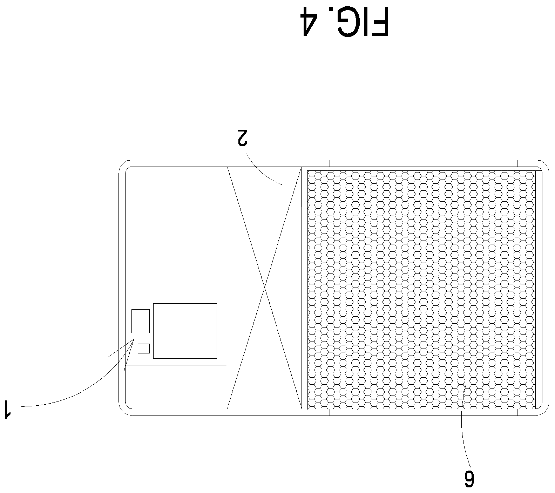

Accordingly, the device for detecting gas and controlling volume of clean gas of the present disclosure includes a gas detection module 1 and a gas guider 2, in which the gas detection module 1 detects a gas to output a gas detection data and converts the gas detection gas into at least one power signal and a PWM signal for providing to the gas guider 2, so that based on the gas detection data, the gas guider 2 is enabled or disabled, and the rotation speed thereof and the output volume of clean gas therefrom are adjusted. Through combining the configuration described above with a filter material 6 as shown in FIG. 4, an air purification device can be formed to monitor the air quality and control the output volume of clean gas according to the air quality, thereby achieving the best benefit of improving and purifying the air quality near a user.

The detailed structure of the gas detection main part 1b of the device for detecting gas and controlling volume of clean gas of the present disclosure is explained as follows.

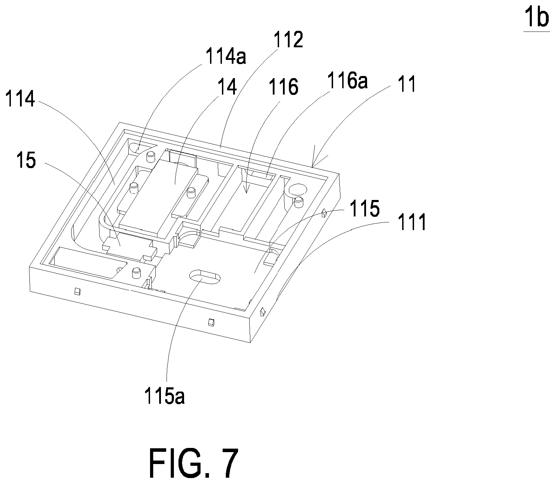

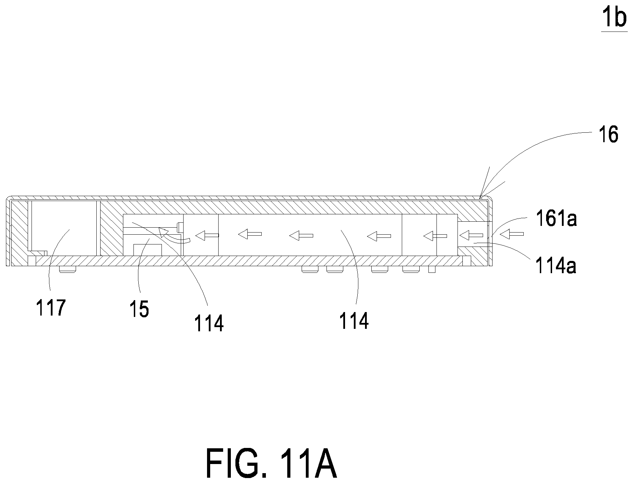

Please refer to FIG. 5, FIGS. 6A to 6C, FIG. 7 and FIGS. 8A to 8B. The gas detection main part 1b above-mentioned includes a base 11, a piezoelectric actuator 12, a driving circuit board 13, a laser component 14, a particulate sensor 15 and an outer cover 16. In the embodiment, the base 11 includes a first surface 111, a second surface 112, a laser loading region 113, a gas-inlet groove 114, a gas-guiding-component loading region 115 and a gas-outlet groove 116. The first surface 111 and the second surface 112 are two surfaces opposite to each other. The laser loading region 113 is hollowed out from the first surface 111 toward the second surface 112. The gas-inlet groove 114 is concavely formed from the second surface 112 and disposed adjacent to the laser loading region 113. The gas-inlet groove 114 includes a gas-inlet 114a which is in communication with the outside the base 11, and is spatially corresponding in position to an inlet opening 161a of the outer cover 16. A transparent window 114b is opened on two lateral walls of the gas-inlet groove 114 and is in communication with the laser loading region 113. Therefore, when the first surface 111 of the base 11 is covered and attached by the outer cover 16, and the second surface 112 is covered and attached by the driving circuit board 13, an inlet path is defined by the gas-inlet groove 114 (as shown in FIG. 7 and FIG. 11A).

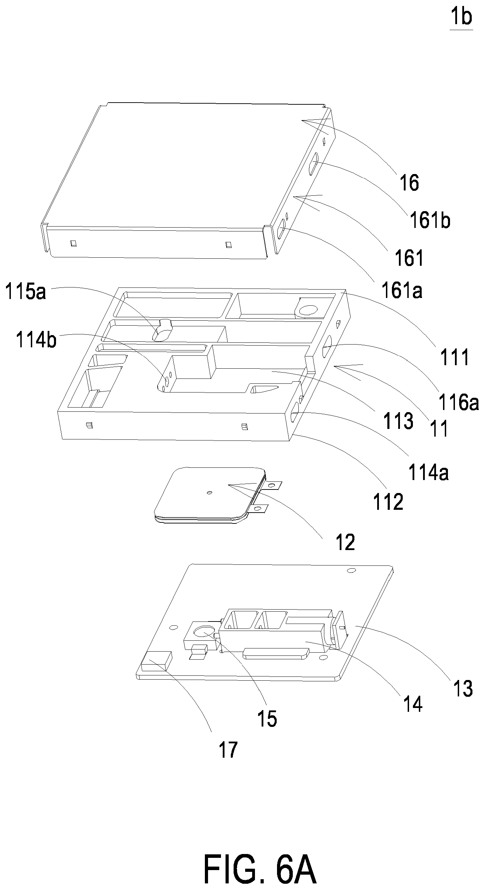

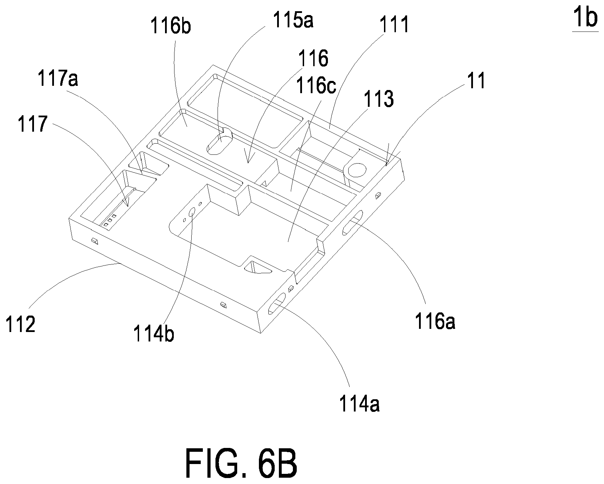

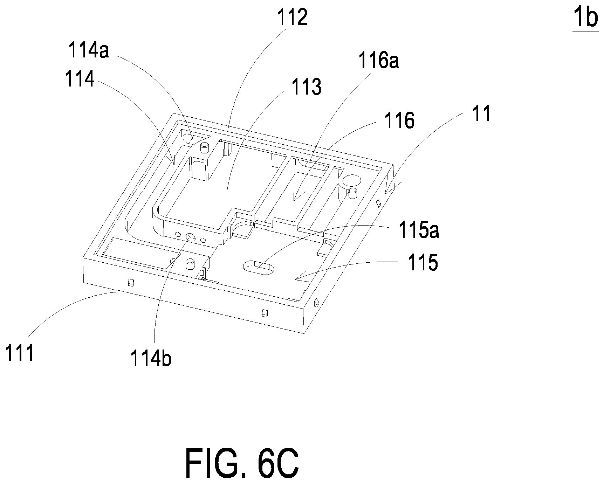

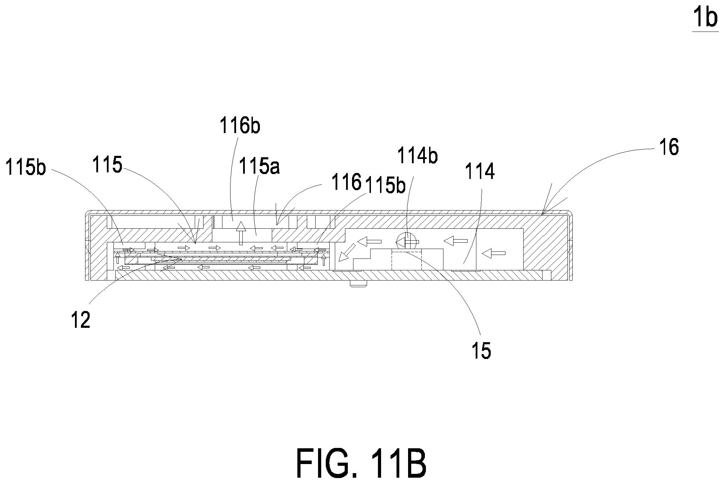

As shown in FIG. 6A to FIG. 6C, the gas-guiding-component loading region 115 mentioned above is concavely formed from the second surface 112 and in communication with the gas-inlet groove 114 and has a ventilation hole 115a penetrating a bottom surface thereof. The gas-outlet groove 116 mentioned above includes a gas-outlet 116a, and the gas-outlet 116a is spatially corresponding to the outlet opening 161b of the outer cover 16. The gas-outlet groove 116 includes a first section 116b and a second section 116c. The first section 116b is concavely formed from the first surface 111 in a region spatially corresponding to a vertical projection area of the gas-guiding-component loading region 115. The second section 116c is hollowed out from the first surface 111 to the second surface 112 in a region where the first surface 111 is misaligned with the vertical projection area of the gas-guiding-component loading region 115 and extended therefrom. The first section 116b and the second section 116c are connected to form a stepped structure. In addition, the first section 116b of the gas-outlet groove 116 is in communication with the ventilation hole 115a of the gas-guiding-component loading region 115, and the second section 116c of the gas-outlet groove 116 is in communication with the gas-outlet 116a. Therefore, when the first surface 111 of the base 11 is attached and covered by the outer cover 16 and the second surface 112 of the base 11 is attached and covered by the driving circuit board 13, an outlet path is defined by the gas-outlet groove 116 (as shown in FIG. 11B to FIG. 11C).

As shown in FIG. 6A and FIG. 7, the laser component 14 and the particulate sensor 15 mentioned above are disposed on the driving circuit board 13 and located within the base 11. In order to clearly describe and illustrate the positions of the laser component 14 and the particulate sensor 15 in the base 11, the driving circuit board 13 is intentionally omitted in FIG. 7. Please refer FIG. 6A, FIG. 7 and FIG. 12, the laser component 14 is accommodated in the laser loading region 113 of the base 11, and the particulate sensor 15 is accommodated in the gas-inlet groove 114 of the base 11 and is aligned to the laser component 14. In addition, the laser component 14 is spatially corresponding to the transparent window 114b. The transparent window 114b is provided for passing therethough a light beam emitted by the laser component 14, so that the light beam enters the gas-inlet groove 114. A light beam path from the laser component 14 passes through the transparent window 114b and extends in an orthogonal direction perpendicular to the gas-inlet groove 114. In the embodiment, the light beam emitted by the laser component 14 passes through the transparent window 114b and enters the gas-inlet groove 114 to irradiate the suspended particles contained in the gas passing through the gas-inlet groove 114. When the suspended particles contained in the gas are irradiated and generate scattered light spots, the scattered light spots are detected and calculated by the particulate sensor 15 to obtain related information about the particle sizes and the concentration of the suspended particles contained in the gas. In one embodiment, the particulate sensor 15 is a PM2.5 sensor.

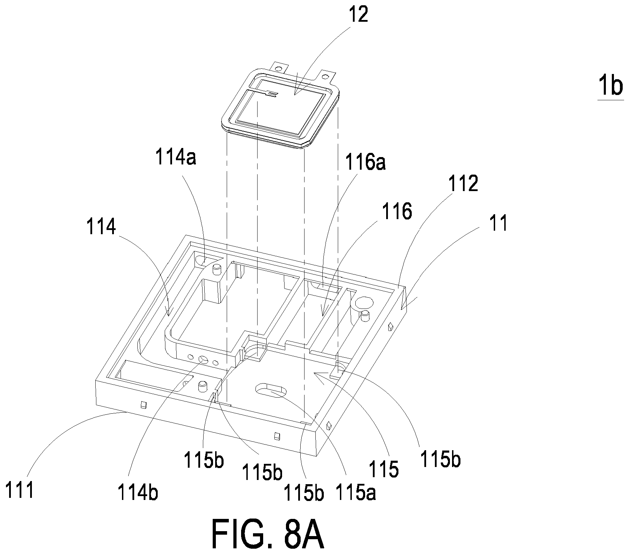

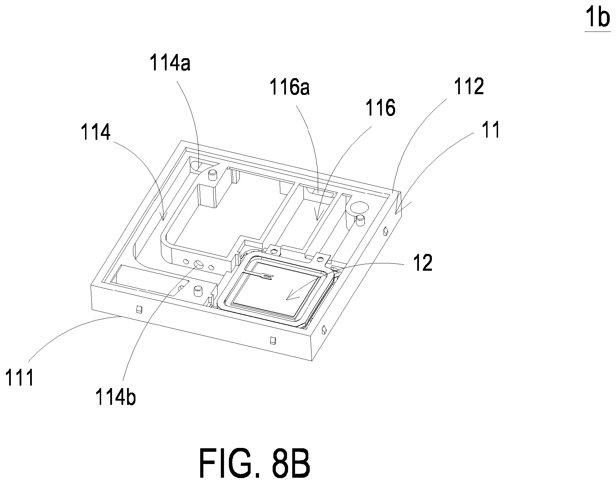

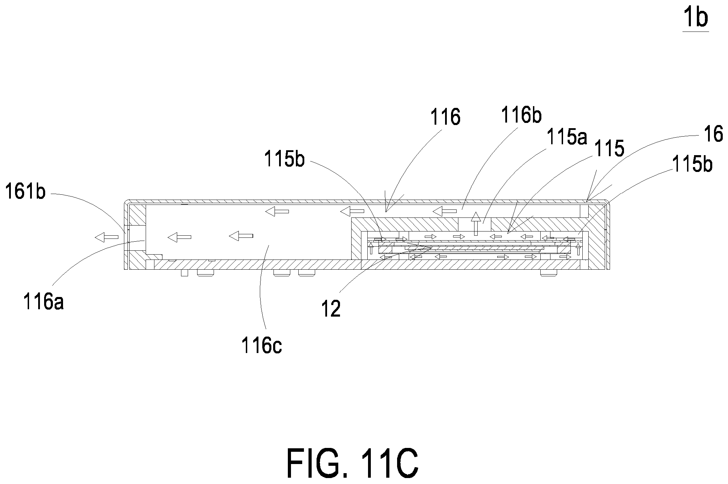

As shown in FIG. 8A, FIG. 8B, FIG. 11B and FIG. 11C, the piezoelectric actuator 12 mentioned above is accommodated in the gas-guiding-component loading region 115 of the base 11. The gas-guiding-component loading region 115 has a square-shape, and four corners of the gas-guiding-component loading region 115 respectively have a positioning protrusion 115b disposed thereon. As shown in FIG. 7, FIG. 11B and FIG. 11C, the gas-guiding-component loading region 115 is in communication with the gas-inlet groove 114. When the piezoelectric actuator 12 is enabled, the gas in the gas-inlet 114 is inhaled into the piezoelectric actuator 12, passes through the ventilation hole 115a of the gas-guiding-component loading region 115, and enters the gas-outlet groove 116.

As shown in FIG. 5 and FIG. 6A, the driving circuit board 13 mentioned above covers the second surface 112 of the base 11. The laser component 14 is positioned and disposed on and electrically connected to the driving circuit board 13. The particulate sensor 15 is also positioned and disposed on and electrically connected to the driving circuit board 13. The outer cover 16 covers the base 11 and includes a side plate 161, wherein the outer cover 16 is covered and attached to the first surface 111 of the base 11, and the side plate 161 has an inlet opening 161a and an outlet opening 161b. When the outer cover 16 covers the base 11, the inlet opening 161a is spatially corresponding to the gas-inlet 114a of the base 11 (as shown in FIG. 11A), and the outlet opening 161b is spatially corresponding to the gas-outlet 116a of the base 11 (As shown in FIG. 11C).

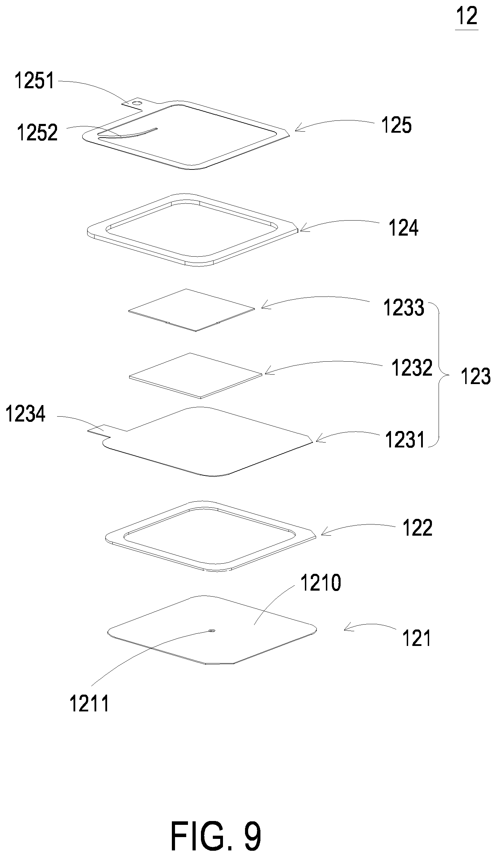

Please refer to FIG. 9, the piezoelectric actuator 12 mentioned above includes a gas-injection plate 121, a chamber frame 122, an actuator element 123, an insulation frame 124 and a conductive frame 125. In the embodiment, the gas-injection plate 121 is made by a flexible material and includes a suspension plate 1210 and a hollow aperture 1211. The suspension plate 1210 is a sheet structure which is permitted to undergo a bending deformation. Preferably but not exclusively, the shape and the size of the suspension plate 1210 are substantially corresponding to the inner edge of the gas-guiding-component loading region 115, but not limited thereto. The shape of the suspension plate 1210 is selected from the group consisting of a square, a circle, an ellipse, a triangle and a polygon. The hollow aperture 1211 penetrates through a center of the suspension plate 1210, so as to allow the gas to flow therethrough. The chamber frame 122 mentioned above is carried and stacked on the gas-injection plate 121, and the shape of the chamber frame 122 is corresponding to the gas-injection plate 121. The actuator element 123 is carried and stacked on the chamber frame 122 so as to collaboratively define a resonance chamber 126 with the chamber frame 122 and the suspension plate 1210 therebetween. The insulation frame 124 is carried and stacked on the actuator element 123 and the appearance of the insulation frame 124 is similar to that of the chamber frame 122. The conductive frame 125 is carried and stacked on the insulation frame 124, and the appearance of the conductive frame 125 is similar to that of the insulation frame 124. The conductive frame 125 includes a conducting pin 1251 and a conducting electrode 1252. The conducting pin 1251 is extended outwardly from an outer edge of the conductive frame 125, and the conducting electrode 1252 is extended inwardly from an inner edge of the conductive frame 125. Moreover, the actuator element 123 further includes a piezoelectric carrying plate 1231, an adjusting resonance plate 1232 and a piezoelectric plate 1233. The piezoelectric carrying plate 1231 is carried and stacked on the chamber frame 122. The adjusting resonance plate 1232 is carried and stacked on the piezoelectric carrying plate 1231. The piezoelectric plate 1233 is carried and stacked on the adjusting resonance plate 1232. The adjusting resonance plate 1232 and the piezoelectric plate 1233 are accommodated in the insulation frame 124. The conducting electrode 1252 of the conductive frame 125 is electrically connected to the piezoelectric plate 1233. In the embodiment, the piezoelectric carrying plate 1231 and the adjusting resonance plate 1232 are made by a conductive material. The piezoelectric carrying plate 1231 includes a piezoelectric pin 1234. The piezoelectric pin 1234 and the conducting pin 1251 are electrically connected to a driving circuit (not shown) on the driving circuit board 13, so as to receive a driving signal (such as a driving frequency and a driving voltage). Moreover, the piezoelectric pin 1234, the piezoelectric carrying plate 1231, the adjusting resonance plate 1232, the piezoelectric plate 1233, the conducting electrode 1252, the conductive frame 125 and the conducting pin 1251 form a circuit loop, in which the insulation frame 124 provides insulation between the conductive frame 125 and the actuator element 123 for avoiding the occurrence of a short circuit, thereby allowing the driving signal to be transmitted to the piezoelectric plate 1233. After receiving the driving signal (the driving frequency and the driving voltage), the piezoelectric plate 1233 deforms due to the piezoelectric effect, and the piezoelectric carrying plate 1231 and the adjusting resonance plate 1232 are further driven to generate the bending deformation in a reciprocating manner. In the embodiment, the adjusting resonance plate 1232 is located between the piezoelectric plate 1233 and the piezoelectric carrying plate 1231 and served as a cushion therebetween, so as to adjust the vibration frequency of the piezoelectric carrying plate 1231. Basically, the thickness of the adjusting resonance plate 1232 is greater than the thickness of the piezoelectric carrying plate 1231, and the vibration frequency of the actuator element 123 can be adjusted by adjusting the thickness of the adjusting resonance plate 1232.

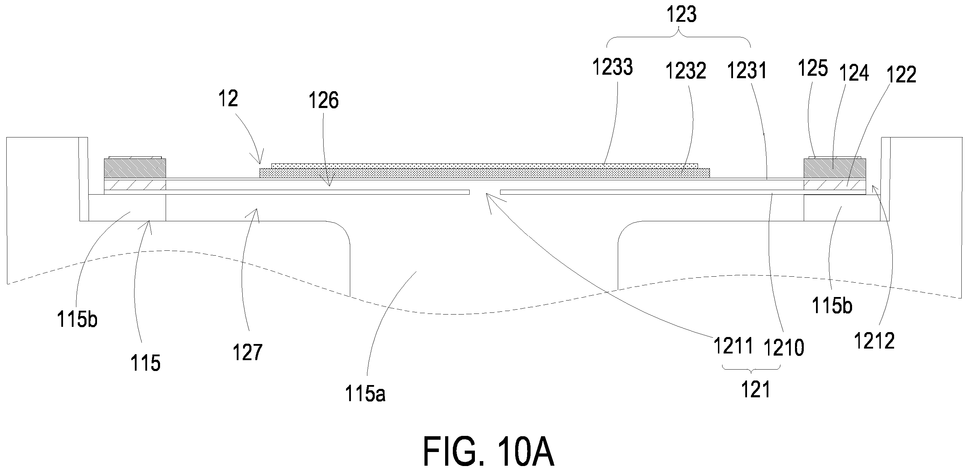

Please refer to FIG. 9 and FIG. 10A. The gas-injection plate 121, the chamber frame 122, the actuator element 123, the insulation frame 124 and the conductive frame 125 are stacked and positioned in the gas-guiding-component loading region 115 sequentially, so that the piezoelectric actuator 12 is disposed and positioned in the gas-guiding-component loading region 115 and supported and positioned on the positioning protrusions 115b. Therefore, a clearance 1212 is defined between the suspension plate 1210 of the piezoelectric actuator 12 and an inner edge of the gas-guiding-component loading region 115 for gas flowing therethrough.

Please refer to FIG. 10A, a flowing chamber 127 is formed between the gas-injection plate 121 and the bottom surface of the gas-guiding-component loading region 115. The flowing chamber 127 is in communication with the resonance chamber 126, which is located between the actuator element 123, the chamber frame 122 and the suspension plate 1210, through the hollow aperture 1211 of the gas-injection plate 121. By controlling the vibration frequency of the gas in the resonance chamber 126 to be close to the vibration frequency of the suspension plate 1210, the Helmholtz resonance effect is generated between the resonance chamber 126 and the suspension plate 1210, so as to improve the efficiency of gas transportation.

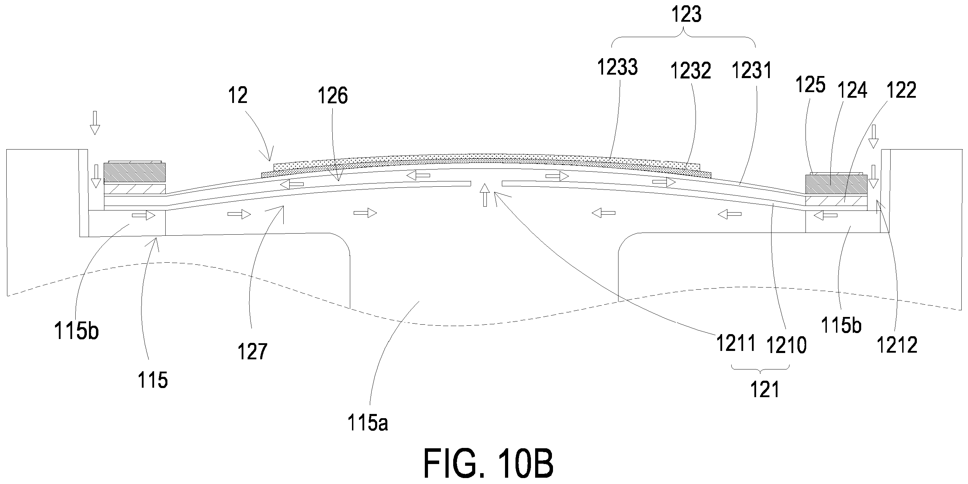

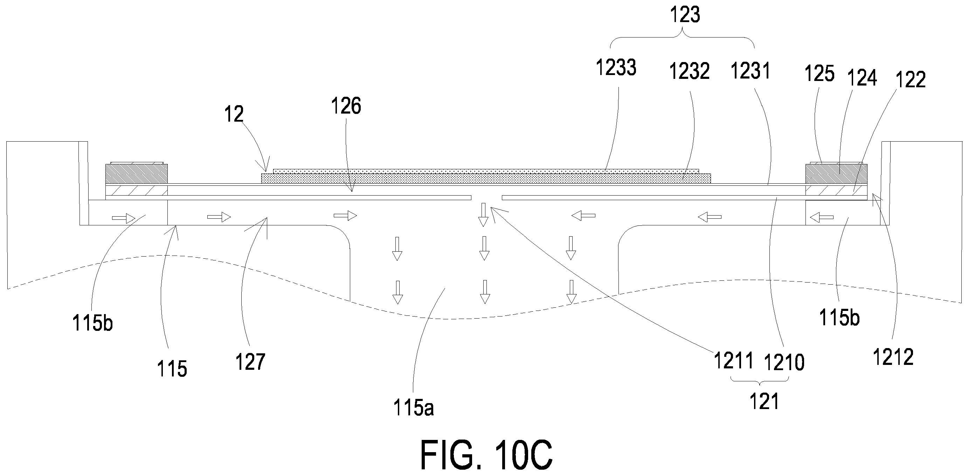

As shown in FIG. 10B, when the piezoelectric plate 1233 is moved away from the bottom surface of the gas-guiding-component loading region 115, the suspension plate 1210 of the gas-injection plate 121 is driven by the piezoelectric plate 1233 to move away from the bottom surface of the gas-guiding-component loading region 115. Therefore, the volume of the flowing chamber 127 is expanded rapidly, the internal pressure of the flowing chamber 127 is decreased to form a negative pressure, and the gas outside the piezoelectric actuator 12 is inhaled through the clearance 1212 and enters the resonance chamber 126 through the hollow aperture 1211. Consequently, the pressure in the resonance chamber 126 is increased to generate a pressure gradient. As shown in FIG. 10C, when the suspension plate 1210 of the gas-injection plate 121 is driven by the piezoelectric plate 1233 to move toward the bottom surface of the gas-guiding-component loading region 115, the gas in the resonance chamber 126 is discharged out rapidly through the hollow aperture 1211, and the gas in the flowing chamber 127 is compressed, so that the converged gas is quickly and massively ejected out under the condition close to an ideal gas state of the Benulli's law and transported to the ventilation hole 115a of the gas-guiding-component loading region 115. Consequently, by repeating the operation steps shown in FIG. 10B and FIG. 10C, the piezoelectric plate 1233 is driven to generate the bending deformation in a reciprocating manner. According to the principle of inertia, since the gas pressure inside the resonance chamber 126 is lower than the equilibrium gas pressure after the converged gas is ejected out, the gas is introduced into the resonance chamber 126 again. Moreover, the vibration frequency of the gas in the resonance chamber 126 is controlled to be close to the vibration frequency of the piezoelectric plate 1233, so as to generate the Helmholtz resonance effect to achieve the gas transportation at high speed and in large quantities.

As shown in FIG. 11A, the gas is inhaled through the inlet opening 161a of the outer cover 16, flows into the gas-inlet groove 114 of the base 11 through the gas-inlet 114a, and is transported to the position of the particulate sensor 15. As shown in FIG. 11B, the piezoelectric actuator 12 is enabled continuously to inhale the gas into the inlet path for facilitating the gas outside the gas detection main part to be introduced rapidly, flow stably, and pass above the particulate sensor 15. At this time, a light beam emitted by the laser component 14 passes through the transparent window 114b and enters the gas-inlet groove 114 to irritate the suspended particles contained in the gas flowing above the particulate sensor 15 in the gas-inlet groove 114. When the suspended particles contained in the gas are irradiated and generate scattered light spots, the scattered light spots are detected and calculated by the particulate sensor 15 for obtaining related information about the particle sizes and the concentration of the suspended particles contained in the gas. Moreover, the gas above the particulate sensor 15 is continuously driven and transported by the piezoelectric actuator 12 so as to flow through the ventilation hole 115a of the gas-guiding-component loading region 115 and enter the first section 116b of the gas-outlet groove 116. At last, as shown in FIG. 11C, after the gas flows into the first section 116b of the gas outlet groove 116, since the gas is continuously transported into the first section 116b by the piezoelectric actuator 12, the gas in the first section 116b is pushed to the second section 11c and discharged through the gas-outlet 116a and the outlet opening 161b.

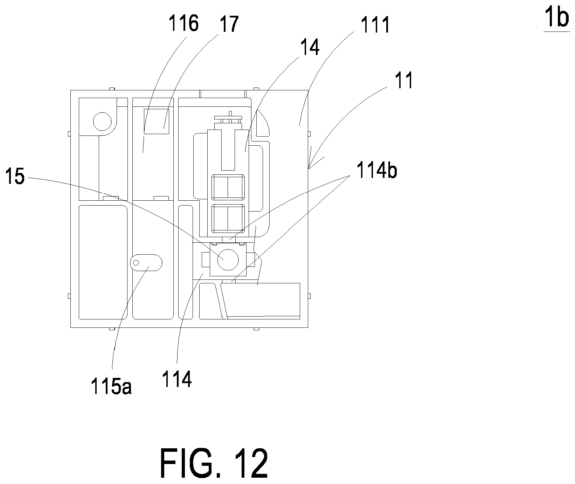

Please refer to FIG. 5 and FIG. 12, the configuration of the gas detection module 1 of the present disclosure can detect not only the existence of the suspended particles in the gas, but also the property of the gas, such as formaldehydes, carbon dioxide, total volatile organic compounds, bacteria, fungi, viruses etc. Therefore, in an embodiment, the gas detection module 1 of the present disclosure further includes a gas sensor 17 positioned and disposed on and electrically connected to the driving circuit board 13 and accommodated in the gas-outlet groove 116 (as shown in FIG. 12), so as to detect the gas introduced into the gas-outlet groove 116.

In conclusion, the present disclosure provides a device for detecting gas and controlling volume of clean gas which combines a gas detection module with at least one gas guider. Through the gas detection module detects a gas to generate a gas detection data and converts the gas detection data into at least one power signal and a pulse-width modulation signal for outputting to the gas guider, the gas guider is enabled or disabled, and the rotation speed thereof and the output volume of clean gas therefrom are adjusted accordingly. Therefore, the air quality can be monitored in real time and the output volume of clean gas can be controlled based on the air quality, thereby achieving a best benefit of improving and purifying the air quality near a user.

## Claims

1. A device for detecting gas and controlling volume of clean gas, comprising:
an external device (5);
a gas detection module (1) comprising a control circuit board (1a), a gas detection main part (1b), a microprocessor (1c) and a communicator (1d), wherein the gas detection main part (1b) is configured to detect a gas to generate a signal and transmits the signal to the microprocessor (1c) for calculating and processing so as to generate a gas detection data, and the microprocessor (1c) is configured to output at least one power signal and a pulse-width modulation (PWM) signal according to the gas detection data, wherein the communicator (1d) is configured to transmit the gas detection data to the external device (5), wherein the communicator (1d) is integrally packaged on and electrically connected to the control circuit board (1a) with the gas detection main part (1b) and the microprocessor (1c), and the microprocessor (1c) is configured to receive, to calculate and to process the signal generated after detecting the gas to generate the gas detection data for providing to the communicator (1d) to perform an external transmission;
at least one gas guider (2) receiving the at least one power signal and the **PWM** signal outputted by the microprocessor (1c), wherein the at least one gas guider (2) is enabled or disabled, as well as a rotation speed and an output volume of clean gas of the at least one gas guider (2) are adjusted according to the at least one power signal and the **PWM** signal; and
at least one second gas detection main part (2b) for detecting and outputting another gas detection data to the external device (5), wherein the external device (5) is configured to receive the another gas detection data and transmits thereof to the microprocessor (1c) via the communicator (1d), and the microprocessor (1c) is configured to receive, to calculate and to process the another gas detection data for outputting the at least one power signal and the **PWM** signal to the at least one gas guider (2), so as to enable or disable the at least one gas guider (2) and adjust the rotation speed thereof and the output volume of clean gas therefrom.

2. The device for detecting gas and controlling volume of clean gas as claimed in claim 1, wherein the gas detection main part (1b) comprises:
a base (11) comprising:
a first surface (111);
a second surface (112) opposite to the first surface (111);
a laser loading region (113) hollowed out from the first surface (111) to the second surface (112);
a gas-inlet groove (114) concavely formed from the second surface (112) and disposed adjacent to the laser loading region (113), wherein the gas-inlet groove (114) comprises a gas-inlet (114a) and a transparent window (114b) opened on two lateral walls thereof and in communication with the laser loading region (113);
a gas-guiding-component loading region (115) concavely formed by the second surface (112) and in communication with the gas-inlet groove (114), and having a ventilation hole (115a) penetrated a bottom surface thereof, wherein each of four corners of the gas-guiding-component loading region (115) respectively comprises a positioning protrusion (115b); and
a gas-outlet groove (116) concavely formed from a region of the first surface (111) spatially corresponding to the bottom surface of the gas-guiding-component loading region (115) and hollowed out from the first surface (111) to the second surface (112) in a region where the first surface (111) is misaligned with the gas-guiding-component loading region (115), wherein the gas-outlet groove (116) is in communication with the ventilation hole (115a) and comprises a gas-outlet (116a) mounted thereon;
a piezoelectric actuator (12) accommodated in the gas-guiding-component loading region (115);
a driving circuit board (13) covering and attaching to the second surface (112) of the base (11);
a laser component (14) positioned on and electrically connected to the driving circuit board (13), and accommodated in the laser loading region (113), wherein a light beam path emitted by the laser component (14) passes through the transparent window (114b) and extends in an orthogonal direction perpendicular to the gas-inlet groove (114);
a particulate sensor (15) positioned on and electrically connected to the driving circuit board (13), and accommodated in the gas-inlet groove (114) at a region in an orthogonal direction perpendicular to the light beam path emitted by the laser component (14) for detecting suspended particles in the air passing through the gas-inlet groove (114) and irradiated by a light beam emitted from the laser component (14); and
an outer cover (16) covering the first surface (111) of the base (11) and comprising a side plate (161), wherein the side plate (161) comprises an inlet opening (16a) and an outlet opening (16b) at positions spatially corresponding to the gas-inlet (114a) and the gas-outlet (116a) of the base (11) respectively, wherein the inlet opening (16a) is spatially corresponding to the gas-inlet (114a) of the base (11) and the outlet opening (16b) is spatially corresponding to the gas-outlet (116a) of the base (11),
wherein the first surface (111) of the base (11) is covered by the outer cover (16), and the second surface (112) of the base (11) is covered by the driving circuit board (13), thereby an inlet path is defined by the gas-inlet groove (114) and an outlet path is defined by the gas-outlet groove (116), by which the piezoelectric actuator (12) introduces the air outside the gas-inlet (114a) of the base (11) into the inlet path defined by the gas-inlet groove (114) through the inlet opening (16a) to pass the particulate sensor (15), the particulate sensor (15) detects a concentration of the suspended particles contained in the air, and the air is guided by the piezoelectric actuator (12) to enter the outlet path defined by the gas-outlet groove (116) through the ventilation hole (115a) and discharged through the gas-outlet (116a) of the base (11) and the outlet opening (16b).

3. The device for detecting gas and controlling volume of clean gas as claimed in claim 2, wherein the piezoelectric actuator (12) comprises:
a gas-injection plate (121) comprising a suspension plate (1210) capable of bending and vibrating and a hollow aperture (1211) formed at a center of the suspension plate (1210);
a chamber frame (122) carried and stacked on the suspension plate (1210);
an actuator element (123) carried and stacked on the chamber frame (122) for bending and vibrating in a reciprocating manner after receiving a voltage;
an insulation frame (124) carried and stacked on the actuator element (123); and
a conductive frame (125) carried and stacked on the insulation frame (124);
wherein the gas-injection plate (121) is supported and positioned on the positioning protrusions (115b) of the gas-guiding-component loading region (115), so that a clearance is defined between the gas-injection plate (121) and an inner edge of the gas-guiding-component loading region (115) for flowing the gas therethrough, a flowing chamber (127) is formed between the gas-injection plate (121) and the bottom surface of the gas-guiding-component loading region (115), and a resonance chamber is collaboratively defined by the actuator element (123), the chamber frame (122) and the suspension plate (1210), by which through driving the actuator element (123) to drive the gas-injection plate (121) to resonate, the suspension plate (1210) of the gas-injection plate (121) generates vibration and displacement in a reciprocating manner, so as to inhale the gas into the flowing chamber (127) through the clearance and then eject out for completing a gas flow transmission.

4. The device for detecting gas and controlling volume of clean gas as claimed in claim 3, wherein the actuator element (123) comprises:
a piezoelectric carrying plate (1231) carried and stacked on the chamber frame (122);
an adjusting resonance plate (1232) carried and stacked on the piezoelectric carrying plate (1231); and
a piezoelectric plate (1233) carried and stacked on the adjusting resonance plate (1232) for driving the piezoelectric carrying plate (1231) and the adjusting resonance plate (1232) to bend and vibrate in a reciprocating manner after receiving the voltage.

5. The device for detecting gas and controlling volume of clean gas as claimed in claim 2, further comprising a gas sensor (17) disposed on and electrically connected to the driving circuit board (13) of the gas detection module (1).

6. The device for detecting gas and controlling volume of clean gas as claimed in claim 5, wherein the gas sensor (17) is a volatile-organic-compound sensor for detecting gas information of carbon dioxide or volatile organic compounds.

7. The device for detecting gas and controlling volume of clean gas as claimed in claim 5, wherein the gas sensor (17) is a formaldehyde sensor for detecting gas information of formaldehyde.

8. The device for detecting gas and controlling volume of clean gas as claimed in claim 5, wherein the gas sensor (17) is a bacteria sensor for detecting information of bacteria or fungi.

9. The device for detecting gas and controlling volume of clean gas as claimed in claim 5, wherein the gas sensor (17) is a virus sensor for detecting information of virus.

10. The device for detecting gas and controlling volume of clean gas as claimed in claim 1, wherein the external device (5) displays the gas detection data and generates a notification alert.

11. The device for detecting gas and controlling volume of clean gas as claimed in claim 10, wherein the external device (5) is a mobile device.

12. The device for detecting gas and controlling volume of clean gas as claimed in claim 1, wherein the microprocessor (1c) transmits the at least one power signal and the PWM signal to the at least one gas guider (2) through a wire (3), and a relay (4) is disposed on the wire (3) to form a buffer switch for achieving a voltage protection for the microprocessor (1c).

13. The device for detecting gas and controlling volume of clean gas as claimed in claim 1, further comprising a filter material (6) for filtering and purifying the gas introduced into the at least one gas guider (2).

14. The device for detecting gas and controlling volume of clean gas as claimed in claim 1, wherein the at least one gas guider (2) is a direct current (DC) fan.

## Patentansprüche

1. Vorrichtung zur Detektion von Gas und zur Steuerung des Volumens von Reingas, umfassend:
eine externe Vorrichtung (5);
ein Gasdetektionsmodul (1), das eine Steuerschaltungsplatine (1a), einen Gasdetektionshauptteil (1b), einen Mikroprozessor (1c) und einen Kommunikator (1d) umfasst, wobei der Gasdetektionshauptteil (1b) konfiguriert ist, um ein Gas zu detektiert, um ein Signal zu erzeugen und um das Signal zwecks Berechnung und Verarbeitung an den Mikroprozessor (1c) zu übertragen, um so Gasdetektionsdaten zu erzeugen, und der Mikroprozessor (1c) konfiguriert ist, um basierend auf den Gasdetektionsdaten mindestens ein Leistungssignal und ein Pulsweitenmodulationssignal (PWM) auszugeben, wobei der Kommunikator (1d) konfiguriert ist, um die Gasdetektionsdaten an die externe Vorrichtung (5) zu übertragen, wobei der Kommunikator (1d) zusammen mit dem Gasdetektionshauptteil (1b) und dem Mikroprozessor (1c) integriert auf der Steuerschaltungsplatine (1a) vorgesehen und elektrisch mit dieser verbunden ist, und der Mikroprozessor (1c) konfiguriert ist, um das nach Detektion des Gases erzeugte Signal zu empfangen, zu berechnen und zu verarbeiten, um die Gasdetektionsdaten zu erzeugen, um dem Kommunikator (1d) zur Durchführung einer externen Übertragung bereitgestellt zu werden;
mindestens einen Gasleiter (2), der das mindestens eine Leistungssignal und das PWM-Signal empfängt, die von dem Mikroprozessor (1c) ausgegeben werden, wobei der mindestens eine Gasleiter (2) aktiviert oder deaktiviert wird, und sowohl die Drehzahl als auch das Ausgabevolumen an Reingas des mindestens einen Gasleiters (2) basierend auf dem mindestens einen Leistungssignal und dem PWM-Signal angepasst werden; und
mindestens einen zweiten Gasdetektionshauptteil (2b) zum Erfassen und Ausgeben weiterer Gasdetektionsdaten an die externe Vorrichtung (5), wobei die externe Vorrichtung (5) konfiguriert ist, um die weiteren Gasdetektionsdaten zu empfangen und diese über den Kommunikator (1d) an den Mikroprozessor (1c) zu übertragen, und der Mikroprozessor (1c) konfiguriert ist, um die weiteren Gasdetektionsdaten zu empfangen, zu berechnen und zu verarbeiten, um das mindestens eine Leistungssignal und das PWM-Signal an den mindestens einen Gasleiter (2) auszugeben, um so den mindestens einen Gasleiter (2) zu aktivieren oder zu deaktivieren, und um dessen Drehzahl sowie das Ausgabevolumen an Reingasluftmenge davon anzupassen.

2. Vorrichtung zur Detektion von Gas und zur Steuerung des Volumens von Reingas nach Anspruch 1, wobei der Gasdetektionshauptteil (1b) umfasst:
eine Basis (11), umfassend:
eine erste Fläche (111);
eine zweite Fläche (112), die der ersten Fläche (111) gegenüberliegt;
eine Laser-Laderegion (113), die bezüglich der ersten Fläche (111) zur zweiten Fläche (112) ausgehöhlt ist;
eine Gaseinlassnut (114), die von der zweiten Fläche (112) konkav ausgebildet und benachbart zur Laser-Laderegion (113) angeordnet ist, wobei die Gaseinlassnut (114) einen Gaseinlass (114a) und ein transparentes Fenster (114b) umfasst, das an zwei lateralen Wänden davon geöffnet ist und mit dem Laser-Laderegion (113) in Verbindung steht;
eine Gasführungskomponenten-Laderegion (115), die durch die zweite Fläche (112) konkav ausgebildet ist und mit der Gaseinlassnut (114) in Verbindung steht, und ein Entlüftungslock (115a) aufweist, die eine Bodenfläche davon durchdringt, wobei jede der vier Ecken der Gasführungskomponenten-Laderegion (115) jeweils einen Positionierungsvorsprung (115b) aufweist; und
eine Gasauslassnut (116), die von einer Region der ersten Fläche (111) konkav ausgebildet ist, die räumlich der Bodenfläche der Gasführungskomponenten-Laderegion (115) entspricht, und von der ersten Fläche (111) zur zweiten Fläche (112) in einer Region ausgehöhlt ist, wo die erste Fläche (111) gegenüber der Gasführungskomponenten-Laderegion (115) versetzt ist, wobei die Gasauslassnut (116) mit dem Entlüftungsloch (115a) in Verbindung steht und einen daran vorgesehenen Gasauslass (116a) aufweist;
einen piezoelektrischen Aktuator (12), der in der Gasführungskomponenten- Laderegion (115) untergebracht ist;
eine Treiberschaltungsplatine (13), die die zweite Fläche (112) der Basis (11) abdeckt und an dieser angebracht ist;
eine Laserkomponente (14), die an der Treiberschaltungsplatine (13) angeordnet und elektrisch damit verbunden ist, sowie in der Laser-Laderegion (113) untergebracht ist, wobei ein von der Laserkomponente (14) emittierter Lichtstrahlpfad durch das transparente Fenster (114b) läuft und sich in einer orthogonalen Richtung senkrecht zur Gaseinlassnut (114) erstreckt;
einen Partikelsensor (15), der an der Treiberschaltungsplatine (13) angeordnet und elektrisch damit verbunden ist, und in der Gaseinlassnut (114) in einer Region in einer orthogonalen Richtung senkrecht zu dem von der Laserkomponente (14) emittierten Lichtstrahlpfad untergebracht ist, um schwebende Partikel in der Luft zu detektieren, die durch die Gaseinlassnut (114) strömt, und von einem von der Laserkomponente (14) emittierten Lichtstrahl bestrahlt wird; sowie
eine äußere Abdeckung (16), die die erste Fläche (111) der Basis (11) abdeckt und eine Seitenplatte (161) umfasst, wobei die Seitenplatte (161) eine Einlassöffnung (16a) und eine Auslassöffnung (16b) an Positionen aufweist, die räumlich dem Gaseinlass (114a) bzw. dem Gasauslass (116a) der Basis (11) entsprechen, wobei die Einlassöffnung (16a) räumlich dem Gaseinlass (114a) der Basis (11) entspricht und die Auslassöffnung (16b) räumlich dem Gasauslass (116a) der Basis (11) entspricht,
wobei die erste Fläche (111) der Basis (11) von der äußeren Abdeckung (16) abgedeckt ist, und die zweite Fläche (112) der Basis (11) von der Treiberschaltungsplatine (13) abgedeckt ist, wodurch ein Einlasspfad durch die Gaseinlassnut (114) definiert ist und ein Auslasspfad durch die Gasauslassnut (116) definiert ist, über den der piezoelektrische Aktuator (12) die Luft außerhalb des Gaseinlasses (114a) der Basis (11) durch die Einlassöffnung (16a) in den durch die Gaseinlassnut (114) definierten Einlasspfad einleitet, um den Partikelsensor (15) zu passieren, wobei der Partikelsensor (15) eine Konzentration der schwebenden Partikel erfasst, die in der Luft enthalten sind, und die Luft durch den piezoelektrischen Aktuator (12) geleitet wird, um durch das Entlüftungsloch (115a) in den durch die Gasauslassnut (116) definierten Auslasspfad einzutreten, und durch den Gasauslass (116a) der Basis (11) sowie die Auslassöffnung (16b) abgeleitet wird.

3. Vorrichtung zur Detektion von Gas und zur Steuerung des Volumens von Reingas nach Anspruch 2, wobei der piezoelektrische Aktuator (12) umfasst:
eine Gasinjektionsplatte (121), die eine Aufhängungsplatte (1210), die sich biegen und schwingen kann, und eine hohle Öffnung (1211) umfasst, die in der Mitte der Aufhängungsplatte (1210) ausgebildet ist;
einen Kammerrahmen (122), der an der Aufhängungsplatte (1210) gelagert und gestapelt ist;
ein Aktuatorelement (123), das an dem Kammerrahmen (122) gelagert und gestapelt ist, um sich nach Empfang einer Spannung in einer oszillierenden Weise zu biegen und zu schwingen;
einen Isolationsrahmen (124), der an dem Aktuatorelement (123) gelagert und gestapelt ist; und
einen konduktiven Rahmen (125), der an dem Isolationsrahmen (124) gelagert und gestapelt ist;
wobei die Gasinjektionsplatte (121) an den Positionierungsvorsprüngen (115b) der Gasführungskomponenten-Laderegion (115) abgestützt und positioniert ist, so dass zwischen der Gasinjektionsplatte (121) und einer inneren Kante der Gasführungskomponenten-Laderegion (115) zum Durchströmen des Gases ein Spalt definiert ist, zwischen der Gasinjektionsplatte (121) und der Bodenfläche der Gasführungskomponenten-Laderegion (115) eine Strömungskammer (127) gebildet ist, und durch das Aktuatorelement (123), den Kammerrahmen (122) und die Aufhängungsplatte (1210) gemeinsam eine Resonanzkammer definiert ist, wodurch durch Ansteuern des Aktuatorelements (123), um die Gasinjektionsplatte (121) in Resonanz zu versetzen, die Aufhängungsplatte (1210) der Gasinjektionsplatte (121) Schwingungen und Verschiebungen in einer oszillierenden Weise erzeugt, um das Gas durch den Spalt in die Strömungskammer (127) anzusaugen und anschließend auszustoßen, um eine vollständige Gasdurchströmung zu bewirken.

4. Vorrichtung zur Detektion von Gas und zur Steuerung des Volumens von Reingas nach Anspruch 3, wobei das Aktuatorelement (123) umfasst:
eine piezoelektrische Trägerplatte (1231), die an dem Kammerrahmen (122) gelagert und gestapelt ist;
eine Einstell-Resonanzplatte (1232), die an der piezoelektrischen Trägerplatte (1231) gelagert und gestapelt ist; und
eine piezoelektrische Platte (1233), die an der Einstell-Resonanzplatte (1232) gelagert und gestapelt ist, um die piezoelektrische Trägerplatte (1231) und die Einstell-Resonanzplatte (1232) nach Empfang der Spannung anzutreiben, sich in oszillierender Weise zu biegen und zu schwingen.

5. Vorrichtung zur Detektion von Gas und zur Steuerung des Volumens von Reingas nach Anspruch 2, ferner umfassend einen Gassensor (17), der an der Treiberschaltungsplatine (13) des Gasdetektionsmoduls (1) angeordnet und elektrisch damit verbunden ist.

6. Vorrichtung zur Detektion von Gas und zur Steuerung des Volumens von Reingas nach Anspruch 5, wobei der Gassensor (17) ein Sensor für flüchtige organische Verbindungen zum Detektieren von Gasinformationen über Kohlendioxid oder flüchtige organische Verbindungen ist.

7. Vorrichtung zur Detektion von Gas und zur Steuerung des Volumens von Reingas nach Anspruch 5, wobei der Gassensor (17) ein Formaldehyd-Sensor zum Detektieren von Gasinformationen über Formaldehyd ist.

8. Vorrichtung zur Detektion von Gas und zur Steuerung des Volumens von Reingas nach Anspruch 5, wobei der Gassensor (17) ein Bakterien-Sensor zum Detektieren von Informationen über Bakterien oder Pilze ist.

9. Vorrichtung zur Detektion von Gas und zur Steuerung des Volumens von Reingas nach Anspruch 5, wobei der Gassensor (17) ein Viren-Sensor zum Detektieren von Informationen über Viren ist.

10. Vorrichtung zur Detektion von Gas und zur Steuerung des Volumens von Reingas nach Anspruch 1, wobei die externe Vorrichtung (5) die Gasdetektionsdaten anzeigt und einen Alarm auslöst.

11. Vorrichtung zur Detektion von Gas und zur Steuerung des Volumens von Reingas nach Anspruch 10, wobei die externe Vorrichtung (5) eine mobile Vorrichtung ist.

12. Vorrichtung zur Detektion von Gas und zur Steuerung des Volumens von Reingas nach Anspruch 1, wobei der Mikroprozessor (1c) das mindestens eine Leistungssignal und das PWM-Signal über eine Leitung (3) an den mindestens einen Gasleiter (2) überträgt, und ein Relais (4) auf der Leitung (3) angeordnet ist, um einen Pufferschalter zum Erzielen eines Spannungsschutzes für den Mikroprozessor (1c) zu bilden.

13. Vorrichtung zur Detektion von Gas und zur Steuerung des Volumens von Reingas nach Anspruch 1, ferner umfassend ein Filtermaterial (6) zum Filtern und Reinigen des in den mindestens einen Gasleiter (2) eingeleiteten Gases.

14. Vorrichtung zur Detektion von Gas und zur Steuerung des Volumens von Reingas nach Anspruch 1, wobei der mindestens eine Gasleiter (2) ein Gleichstrom-Lüfter (DC-Lüfter) ist.

## Revendications

1. Dispositif de détection de gaz et de commande de volume de gaz propre, comprenant:
un dispositif externe (5);
un module de détection de gaz (1) comprenant une carte de circuit de commande (1a), une partie principale de détection de gaz (1b), un microprocesseur (1c) et un communicateur (1d), dans lequel la partie principale de détection de gaz (1b) est configurée pour détecter un gaz afin de générer un signal et transmet le signal au microprocesseur (1c) pour calcul et traitement de manière à générer des données de détection de gaz, et le microprocesseur (1c) est configuré pour délivrer en sortie au moins un signal de puissance et un signal de modulation en largeur d'impulsion (PWM) en fonction des données de détection de gaz, dans lequel le communicateur (1d) est configuré pour transmettre les données de détection de gaz au dispositif externe (5), dans lequel le communicateur (1d) est intégralement encapsulé sur la carte de circuit de commande (1a), et connecté électriquement à celle-ci, avec la partie principale de détection de gaz (1b) et le microprocesseur (1c), et le microprocesseur (1c) est configuré pour recevoir, calculer et traiter le signal généré après détection du gaz pour générer les données de détection de gaz afin de les fournir au communicateur (1d) pour effectuer une transmission externe;
au moins un dispositif de guidage de gaz (2) recevant l'au moins un signal de puissance et le signal PWM délivrés en sortie par le microprocesseur (1c), dans lequel l'au moins un dispositif de guidage de gaz (2) est activé ou désactivé, et de même une vitesse de rotation et un volume de sortie de gaz propre de l'au moins un dispositif de guidage de gaz (2) sont ajustés en fonction de l'au moins un signal de puissance et du signal PWM; et
au moins une seconde partie principale de détection de gaz (2b) pour détecter et délivrer en sortie d'autres données de détection de gaz vers le dispositif externe (5), dans lequel le dispositif externe (5) est configuré pour recevoir les autres données de détection de gaz et transmet celles-ci au microprocesseur (1c) via le communicateur (1d), et le microprocesseur (1c) est configuré pour recevoir, calculer et traiter les autres données de détection de gaz pour délivrer en sortie l'au moins un signal de puissance et le signal PWM vers l'au moins un dispositif de guidage de gaz (2), de manière à activer ou désactiver l'au moins un dispositif de guidage de gaz (2) et à ajuster sa vitesse de rotation et le volume de sortie de gaz propre provenant de celui-ci.

2. Dispositif de détection de gaz et de commande de volume de gaz propre selon la revendication 1, dans lequel la partie principale de détection de gaz (1b) comprend:
une base (11) comprenant:
une première surface (111);
une seconde surface (112) opposée à la première surface (111);
une région de chargement laser (113) évidée de la première surface (111) à la seconde surface (112);
une rainure d'entrée de gaz (114) formée de manière concave à partir de la seconde surface (112) et disposée à proximité adjacente de la région de chargement laser (113), dans lequel la rainure d'entrée de gaz (114) comprend une entrée de gaz (114a) et une fenêtre transparente (114b) ouverte sur deux parois latérales de celle-ci et en communication avec la région de chargement laser (113);
une région de chargement de composant de guidage de gaz (115) formée de manière concave par la seconde surface (112) et en communication avec la rainure d'entrée de gaz (114), et ayant un trou de ventilation (115a) pénétrant dans une surface inférieure de celle-ci, dans lequel chacun des quatre coins de la région de chargement de composant de guidage de gaz (115) comprend respectivement une saillie de positionnement (115b); et
une rainure de sortie de gaz (116) formée de manière concave à partir d'une région de la première surface (111), correspondant spatialement à la surface inférieure de la région de chargement de composant de guidage de gaz (115), et évidée de la première surface (111) à la seconde surface (112) dans une région où la première surface (111) est mal alignée avec la région de chargement de composant de guidage de gaz (115), dans lequel la rainure de sortie de gaz (116) est en communication avec le trou de ventilation (115a) et comprend une sortie de gaz (116a) montée dessus;
un actionneur piézoélectrique (12) logé dans la région de chargement de composant de guidage de gaz (115);
une carte de circuit imprimé d'excitation (13) recouvrant la seconde surface (112) de la base (11) et fixée à ladite surface;
un composant laser (14) positionné sur la carte de circuit imprimé d'excitation (13), et connecté à celle-ci, et logé dans la région de chargement laser (113), dans lequel un trajet de faisceau lumineux émis par le composant laser (14) passe à travers la fenêtre transparente (114b) et s'étend dans une direction orthogonale perpendiculaire à la rainure d'entrée de gaz (114);
un capteur de particules (15) positionné sur la carte de circuit imprimé d'excitation (13), et connecté électriquement à celle-ci, et logé dans la rainure d'entrée de gaz (114) au niveau d'une région dans une direction orthogonale perpendiculaire au trajet de faisceau lumineux émis par le composant laser (14) pour détecter des particules en suspension dans l'air passant à travers la rainure d'entrée de gaz (114) et irradiées par un faisceau lumineux émis par le composant laser (14); et
un couvercle externe (16) recouvrant la première surface (111) de la base (11) et comprenant une plaque latérale (161), dans lequel la plaque latérale (161) comprend une ouverture d'entrée (16a) et une ouverture de sortie (16b) à des positions correspondant spatialement à l'entrée de gaz (114a) et à la sortie de gaz (116a) de la base (11) respectivement, dans lequel l'ouverture d'entrée (16a) correspond spatialement à l'entrée de gaz (114a) de la base (11) et l'ouverture de sortie (16b) correspond spatialement à la sortie de gaz (116a) de la base (11),
dans lequel la première surface (111) de la base (11) est recouverte par le couvercle externe (16), et la seconde surface (112) de la base (11) est recouverte par la carte de circuit imprimé d'excitation (13), un trajet d'entrée est ainsi défini par la rainure d'entrée de gaz (114) et un trajet de sortie est ainsi défini par la rainure de sortie de gaz (116), par laquelle l'actionneur piézoélectrique (12) introduit l'air à l'extérieur de l'entrée de gaz (114a) de la base (11) dans le trajet d'entrée défini par la rainure d'entrée de gaz (114) à travers l'ouverture d'entrée (16a) pour traverser le capteur de particules (15), le capteur de particules (15) détecte une concentration des particules en suspension contenues dans l'air, et l'air est guidé par l'actionneur piézoélectrique (12) pour entrer dans le trajet de sortie défini par la rainure de sortie de gaz (116) à travers le trou de ventilation (115a) et évacué à travers la sortie de gaz (116a) de la base (11) et l'ouverture de sortie (16b).

3. Dispositif de détection de gaz et de commande de volume de gaz propre selon la revendication 2, dans lequel l'actionneur piézoélectrique (12) comprend:
une plaque d'injection de gaz (121) comprenant une plaque de suspension (1210) apte à se courber et à vibrer et une ouverture creuse (1211) formée au niveau d'un centre de la plaque de suspension (1210);
un cadre de chambre (122) porté et empilé sur la plaque de suspension (1210);
un élément actionneur (123) porté et empilé sur le cadre de chambre (122) pour se courber et vibrer d'une manière alternée après avoir reçu une tension;
un cadre d'isolation (124) porté et empilé sur l'élément actionneur (123); et
un cadre conducteur (125) porté et empilé sur le cadre d'isolation (124);
dans lequel la plaque d'injection de gaz (121) est supportée et positionnée sur les saillies de positionnement (115b) de la région de chargement de composant de guidage de gaz (115), de sorte qu'un jeu est défini entre la plaque d'injection de gaz (121) et un bord interne de la région de chargement de composant de guidage de gaz (115) pour l'écoulement du gaz à travers celui-ci, une chambre d'écoulement (127) est formée entre la plaque d'injection de gaz (121) et la surface inférieure de la région de chargement de composant de guidage de gaz (115), et une chambre de résonance est définie de manière collaborative par l'élément actionneur (123), le cadre de chambre (122) et la plaque de suspension (1210), par laquelle, par excitation de l'élément actionneur (123) pour exciter la plaque d'injection de gaz (121) pour qu'elle résonne, la plaque de suspension (1210) de la plaque d'injection de gaz (121) génère des vibrations et un déplacement d'une manière alternée, de manière à aspirer le gaz pour l'amener dans la chambre d'écoulement (127) à travers le jeu puis à l'éjecter pour achever une transmission de flux de gaz.

4. Dispositif de détection de gaz et de commande de volume de gaz propre selon la revendication 3, dans lequel l'élément actionneur (123) comprend:
une plaque de soutien piézoélectrique (1231) portée et empilée sur le cadre de chambre (122);
une plaque de résonance d'ajustement (1232) portée et empilée sur la plaque de soutien piézoélectrique (1231); et
une plaque piézoélectrique (1233) portée et empilée sur la plaque de résonance d'ajustement (1232) pour exciter la plaque de support piézoélectrique (1231) et la plaque de résonance d'ajustement (1232) pour qu'elles se courbent et vibrent d'une manière alternée après réception de la tension.

5. Dispositif de détection de gaz et de commande de volume de gaz propre selon la revendication 2, comprenant en outre un capteur de gaz (17) disposé sur la carte de circuit imprimé d'excitation (13) du module de détection de gaz (1), et connecté électriquement à celle-ci.

6. Dispositif de détection de gaz et de commande de volume de gaz propre selon la revendication 5, dans lequel le capteur de gaz (17) est un capteur de composés organiques volatils pour détecter des informations de gaz de dioxyde de carbone ou de composés organiques volatils.

7. Dispositif de détection de gaz et de commande de volume de gaz propre selon la revendication 5, dans lequel le capteur de gaz (17) est un capteur de formaldéhyde pour détecter des informations de gaz de formaldéhyde.

8. Dispositif de détection de gaz et de commande de volume de gaz propre selon la revendication 5, dans lequel le capteur de gaz (17) est un capteur de bactéries pour détecter des informations de bactéries ou de champignons.

9. Dispositif de détection de gaz et de commande de volume de gaz propre selon la revendication 5, dans lequel le capteur de gaz (17) est un capteur de virus pour détecter des informations de virus.

10. Dispositif de détection de gaz et de commande de volume de gaz propre selon la revendication 1, dans lequel le dispositif externe (5) affiche les données de détection de gaz et génère une alerte de notification.

11. Dispositif de détection de gaz et de commande de volume de gaz propre selon la revendication 10, dans lequel le dispositif externe (5) est un dispositif mobile.

12. Dispositif de détection de gaz et de commande de volume de gaz propre selon la revendication 1, dans lequel le microprocesseur (1c) transmet l'au moins un signal de puissance et le signal PWM à l'au moins un dispositif de guidage de gaz (2) par le biais d'un fil (3), et un relais (4) est disposé sur le fil (3) pour former un commutateur tampon pour obtenir une protection de tension pour le microprocesseur (1c).

13. Dispositif de détection de gaz et de commande de volume de gaz propre selon la revendication 1, comprenant en outre un matériau filtrant (6) pour filtrer et purifier le gaz introduit dans l'au moins un dispositif de guidage de gaz (2).

14. Dispositif de détection de gaz et de commande de volume de gaz propre selon la revendication 1, dans lequel l'au moins un dispositif de guidage de gaz (2) est un ventilateur à courant continu (CC).
